(19) Europäisches Patentamt
European Patent Office
Office européen des brevets

(11) Publication number: **0 071 227**
**B1**

(12) **EUROPEAN PATENT SPECIFICATION**

(45) Date of publication of patent specification: **11.11.87**

(21) Application number: **82106738.6**

(22) Date of filing: **26.07.82**

(51) Int. Cl.$^4$: **C 07 D 487/04,**
C 07 D 495/04,
C 07 D 491/04, A 61 K 31/505
// C07D493/04 ,(C07D487/04,
239:00, 209:00),(C07D495/04,
333:00, 239:00),(C07D491/04,
307:00, 239:00)

(54) **Anti-leukemic beta-glycosyl C-nucleosides.**

(30) Priority: **31.07.81 US 288848**

(43) Date of publication of application:
**09.02.83 Bulletin 83/06**

(45) Publication of the grant of the patent:
**11.11.87 Bulletin 87/46**

(84) Designated Contracting States:
**DE FR GB IT**

(56) References cited:

**TETRAHEDRON LETTERS, vol. 21, 1980, Pergamon Press Ltd., GB, pages 1013-1016, MU-Ill LIM et al.: "Synthesis of the Pyrrolo (3,2-d) Pyrimidine C-Nucleoside Isostere of Inosine1"**

**TETRAHEDRON LETTERS, vol. 22, 1981, Pergamon Press Ltd., GB, pages 25-28, MU-Ill LIM et al.: "Synthesis of "9-Deazaadenosine"; A new Cytotoxic C-Nucleoside Isostere of Adenosine"**

**The file contains technical information submitted after the application was filed and not included in this specification**

(73) Proprietor: **Sloan-Kettering Institute For Cancer Research**
**1275 York Avenue**
**New York New York 10021 (US)**

(72) Inventor: **Klein, Robert S.**
**108 Bradford Avenue**
**Rye New York (US)**
Inventor: **Lim, Mu-ill**
**395 Westchester Avenue**
**Port Chester New York (US)**
Inventor: **Ren, Wuyun**
**130 Pelham Road**
**New Rochelle New York (US)**
Inventor: **Burchenal, Joseph H.**
**Juniper Road**
**Noroton Connecticut (US)**

(74) Representative: **Patentanwälte Schulze Horn und Hoffmeister**
**Goldstrasse 36**
**D-4400 Münster (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to beta-glycosyl C-Nucleoside compounds which are able to inhibit the growth of leukemic cells, according to the species defined in claim 1.

Methods for making N-Nucleosides similar to the C-Nucleosides, which are the subject of this invention, are well known and well practiced. These techniques basically involve fusing an appropriate sugar to an appropriate base to form the required compound. These techniques cannot be applied to C-Nucleosides because of the low reactivity of the carbon site when compared with the nitrogen site.

To form C-Nucleosides, it has been found convenient to start with an appropriately substituted sugar moiety and "build" the desired base. However, known schemes for accomplishing this are difficult to follow and have only limited applicability because of the reagents used (see for example Gupta et al Abstract No. 40, 175 A. C. S. National meeting, Anaheim, California, March 13-17, 1978). Another procedure was also applied to the synthesis of 9-deazainosine in Lim et al., Tetrahedron Letters, Vol. 21, pp. 1013-1016 (1980). However, this compound has been found to have essentially no antitumor activity. For comparison, this compound is included in Table 1 of the patent description showing relative inhibition activity for various inventive compounds (X=NH, $R_1$=OH). A similar procedure was applied to synthesise 9-deazainosine according to Lim and Klein, Tetrahedron Letters, Vol. 22, pp. 25-28 (1981). This compound shows activity many orders of magnitude greater than 9-deazainosine as evidenced in Table 1.

It is the object of the invention to supply further potent new beta-glycosyl C-nucleoside compounds which exhibit activity against leukemia.

This object is solved by beta-glycosyl C-nucleoside compounds of the following formula:

Beta-glycosyl C-Nucleoside compounds of the formula

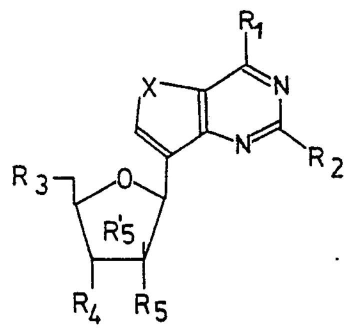

wherein

X is S or O

$R_6$
/
$R_1$ is N , or $SR_8$, or $OR_8$
\
$R_7$

wherein $R_6$, $R_7$ and $R_8$ are independently selected from H or alkyl of 1 to 6 carbon atoms; or

$R_2$ is H,
$R_3$ is OH,
$R_4$ is OH,
$R_5$ is OH or H,
$R'_5$ is H.

Further subclaims 2—5 refer to especially useful compounds.

The beta-glycosyl C-nucleoside compounds of the present invention have been shown to have anti-leukemic activity using usual techniques of the art. The results of these tests were summarized in Table 1. In addition, *in vivo* antitumor activity has been shown as summarized in Table 2. It is noted that the first compound in Table 1 is the compound reported in Lim et al (supra). This compound shows a lack of activity. Similar tests were also run on certain alpha-glycosyl nucleosides analogous to the inventive beta-glycosyl compounds, however these were found to be without activity.

The following preparative examples illustrate the presently preferred method of synthesizing the compounds of the present invention. (By lower alkyls is meant straight or branched alkyls of up to 6 carbon atoms.)

The invention therefor relates also to a method as claimed in claim 6

Preparative Example for Thieno (3,2-d)-(and Furo (3,2-d)) Pyrimidine Adenosine Analogs

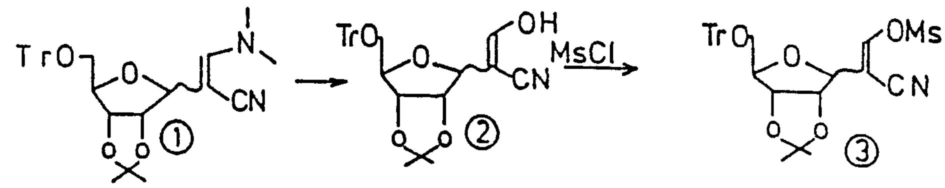


3-Methanesulfonyloxy-2-(2'3'-0-isopropylidene-5'-0-trityl-D-ribofuranosyl)-acrylonitrile

Hydrolysis of 24 g (47 mmol) of dimethylamino acrylonitrile ① as described previously afforded the corresponding crude 2-formyl acetonitrile derivative ②. Without purification ② was dissolved in 160 ml of chloroform containing 8.30 ml of triethylamine and treated dropwise with a solution of 4.22 ml (54 mmol) of methanesulfonyl chloride in 160 ml of chloroform at 0°C with efficient stirring. After one hour at 0°C the reaction mixture was diluted to 500 ml with chloroform and washed well with brine. The organic layer was dried over sodium sulfate and evaporated to dryness to give a crude anomeric mixture of ③ obtained as a foam (22 g). Purification and separation by chromatography of a small sample afforded pure anomers which were readily identified by NMR spectroscopy. The crude material was of satisfactor purity to be utilized directly in the following step.

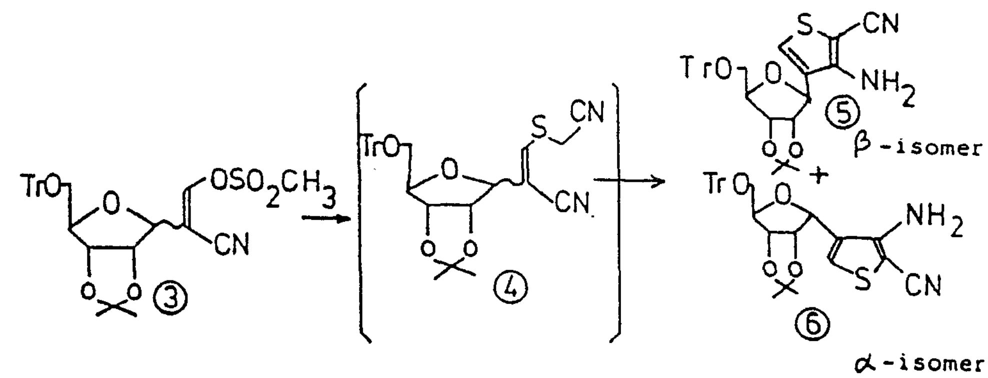


4-(2',3'-O-Isopropylidene-5'-O-trityl-β-(and α-) D-ribofuranosyl)-3-amino-2-cyanothiophene ⑤ and ⑥

To a solution of intermediate ③ (20 g, obtained from the previous step) in 430 ml of absolute ethanol was added acetylthioacetonitrile (8 g, 70 mmole). The reaction mixture was heated to reflux under $N_2$ for 7 hours and evaporated to dryness *in vacuo*. The residue was partitioned between chloroform and water (300 ml each) and the organic layer washed again with water. The chloroform solution was then dried over anhydrous sodium sulfate and evaporated to dryness *in vacuo*. The residue containing the α-β-anomeric mixture of ⑤ and ⑥ was purified by column chromatography on silica gel (Toluene) to give 5.6 g (24.5% from ① in the previous step) of the 3-amino-2-cyanothiophene β-C-nucleoside ⑤ and 2.5 g (11% from ①) of the corresponding α-C-nucleoside ⑥. Both were obtained as syrup. Their structure was confirmed by elemental analysis and NMR spectroscopy.

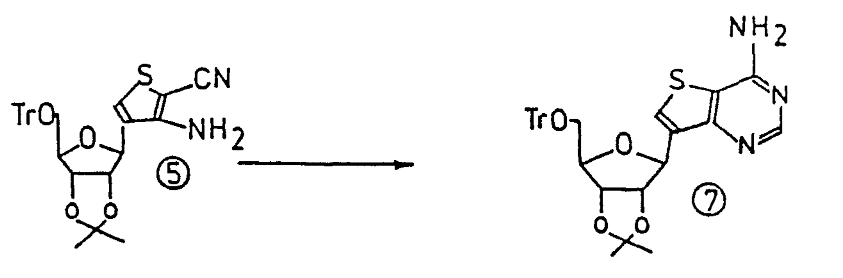

7-(2',3'-0-Isopropylidene-5'-0-trityl-β-D-ribofuranosyl)-4-amino-thieno(3,2-d)pyrimidine ⑦.

A solution of ⑤ (1.2 g, 2.2 mmol) in 30 ml of absolute ethanol was heated to reflux and to this was added in several portions 7 g of formamidine acetate (67 mmol) over a period of 7 days. The solvent was then removed *in vacuo* and the residue extracted with chloroform. The chloroform solution was then washed with water, dried over anhydrous sodium sulfate and evaporated to dryness. The crude material containing ⑦ was purified by column chromatography on silica gel (chloroform-methanol: 20/1) to give the blocked thieno (3, 3-*d)* pyrimidine ⑦ (1.01 g, 80% from ⑤) as a foam. The structure was confirmed by elemental analysis and NMR spectroscopy.

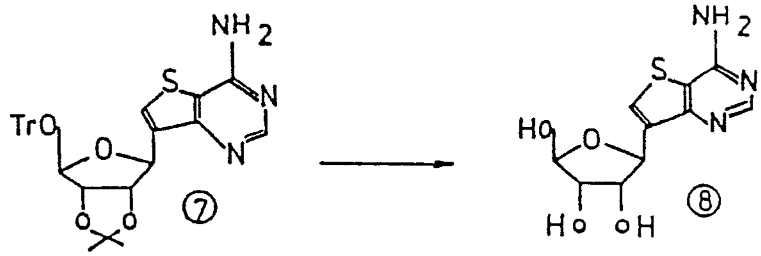

7-(β-D-ribofuranosyl-4-amino-thieno(3,2-d)pyrimidine ⑧ (claim 2)

A mixture of the blocked C-nucleoside ⑦ (200 mg, 0.35 mmol) and 4 ml of a 12% solution of hydrogen chloride in methanol was stirred at room temperature for 10 minutes. Diethylether (15 ml) was then gradually added to precipitate ⑧ as an amorphous solid which slowly crystallizes. Filtration and washing with diethylether finally affords 98 mg (85%) of ⑧ as a dihydrochloride salt m.p. 154-155°C.

Elemental Analysis Calculated for: C: 37.08; H: 4.24; N: 11.79; S: 8.99; Cl: 19.90.
Found: C: 37.74; H: 4.17; N: 11.89; S: 9.30; Cl: 20.40.

The structure was also confirmed by NMR spectroscopy.

The furo (3,2-*d*) pyrimidine compounds are prepared in an analogous manner, as shown by the following reaction scheme when compared with this preparative example.

SEQUENCE FOR THE FURO [3, 2 *d*] PYRIMIDINE ADENOSINE ANALOG

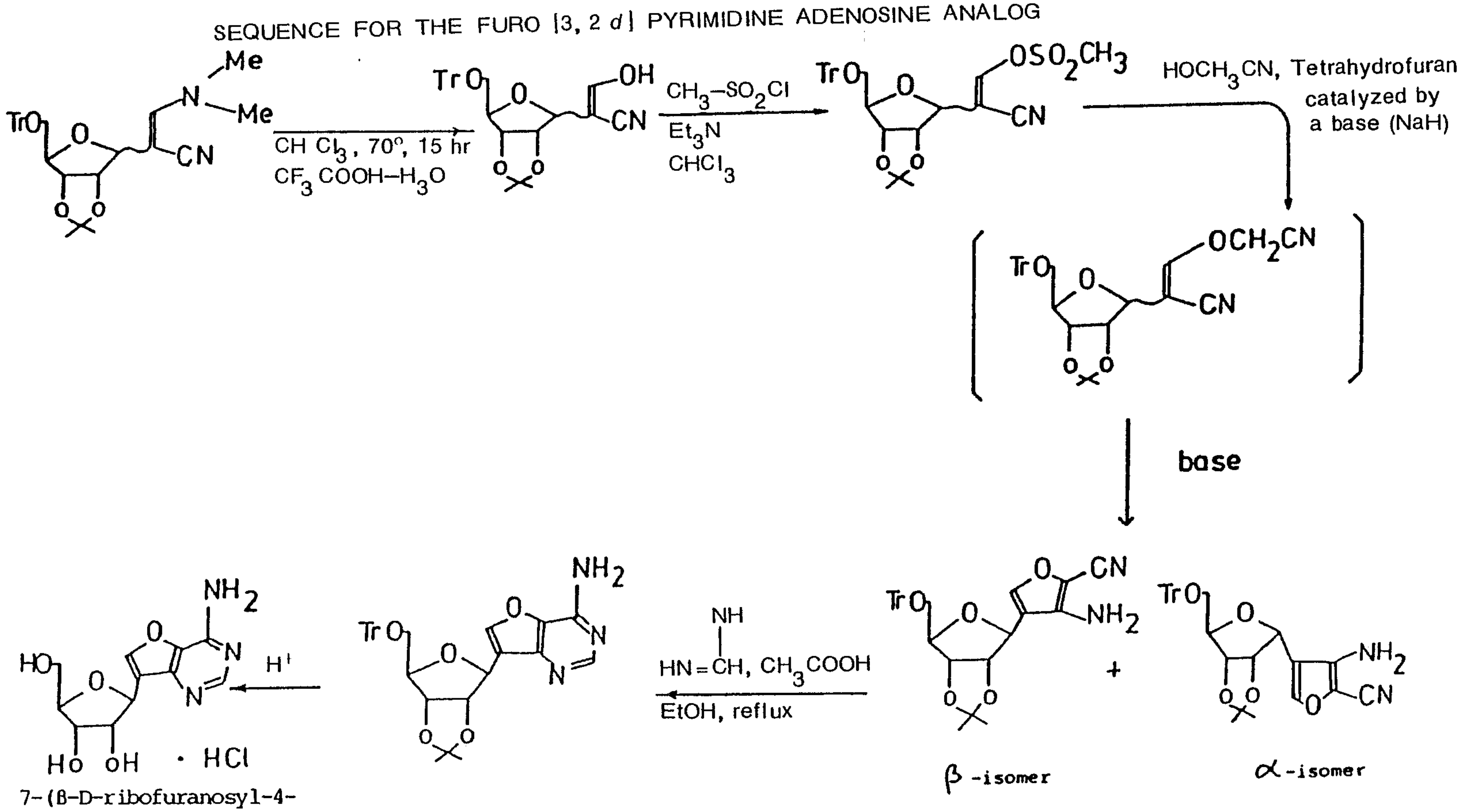


β-isomer

α-isomer

7-(B-D-ribofuranosyl-4-
amino-furo(3,2-d)pyrimidine

By utilization of the general procedure (J. Am. Chem. Soc., 1981 (103 pp 932-933)) for conversion of ribonucleosides to 2′-deoxynucleosides the following
7-(2′-Deoxy-β-D-ribofuranosyl)-4-amino-thieno(3,2-d)pyrimidine (claim 3)
7-(2′-Deoxy-β-D-ribofuranosyl)-4-amino-furo(3,2-d)pyrimidine (claim 5)

Preparation of thieno (3,2-d) pyrimidine C-nucleoside derivatives

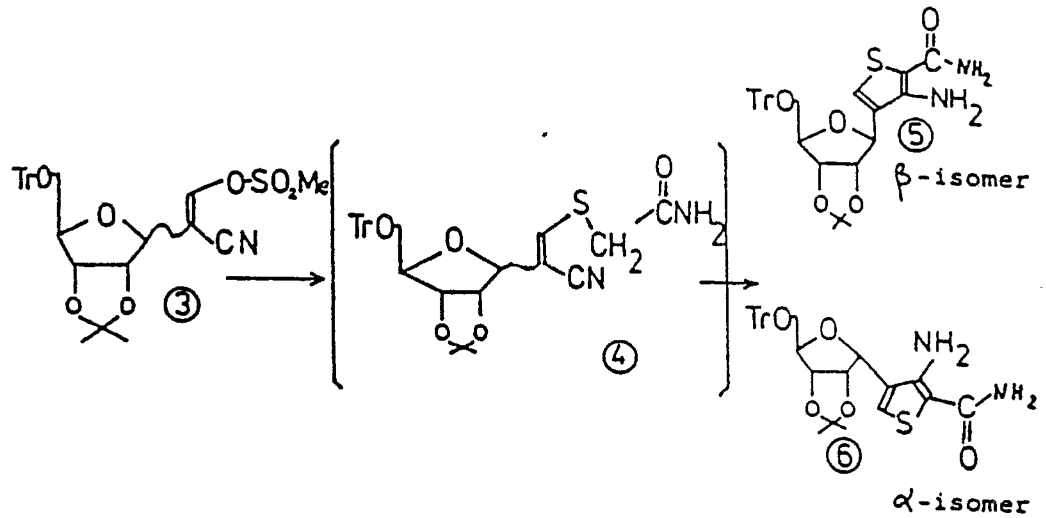


4-(2′,3′-0-Isopropylidene-5′-0-trityl-β-(and -) D-ribofuranosyl)-3-amino-2-carboxamido-thiophene ⑤ and ⑥.

To a suspension of 3-mesyloxy acetonitrile ③ (6 g, 10.7 mmol, prepared by the method above described) in 180 ml of absolute ethanol was added mercaptocetamide (1.5 g, 16.4 mmol) and anhydrous sodium carbonate (1.7 g, 16.03 mmol). The reaction mixture was heated to reflux with stirring for 18 hours under a nitrogen atmosphere, allowed to cool to room temperature and filtered. The filtrate was evaporated to dryness *in vacuo* and the residue containing the isomers ⑤ and ⑥ chromatographed on a column of silica gel with toluene-ethyl acetate (20:1). This separation afforded pure 3-amino-2-carboxamido-β-isomer ⑤ as a foam (2.62 g, 40% from ③ and the pure α-isomer ⑥ also a foam (2.94 g, 45% from ③). The structure of each was confirmed by elemental analysis and NMR spectroscopy.

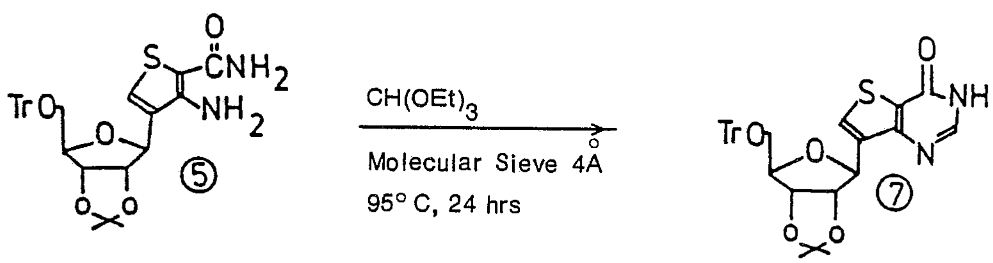


7-(2′,3′-0-Isopropylidene-5′-0-trityl-β-D-ribofuranosyl)-3H-4-oxo-thieno(3,2-d)-pyrimidine ⑦

To a suspension of 3-amino-2-carboxamido-thiophene ⑤ (3.5 g, 6.29 mmol) in 20 ml of triethylorthoformate was added 1 g of finely ground molecular sieve (4Å). The reaction mixture was heated at 95°C and stirred for 24 hours. After cooling to room temperature, it was filtered and to the clear filtrate was added 10 ml of petroleum ether (40-60°C) to precipitate compound ⑦ as a solid. This was collected by filtration, pressed into a cake, washed with petroleum ether and dried *in vacuo.* This procedure afforded ⑦ (2 g, 56%) as a white powder, mp 128-130°C.

The structure was confirmed by elemental analysis and by NMR spectroscopy.

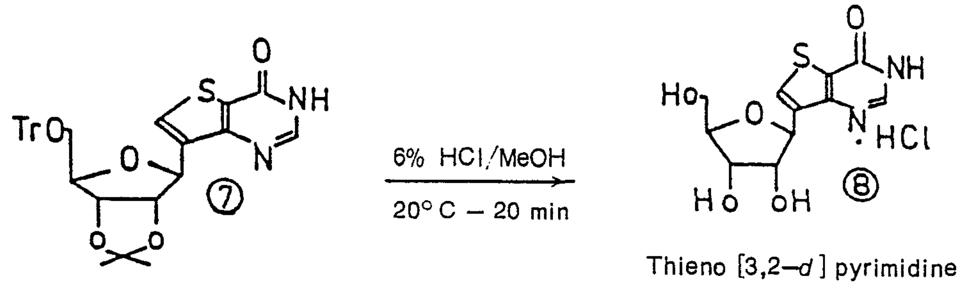


Thieno [3,2–*d*] pyrimidine
analog of INOSINE

7-(β-D-Ribofuranosyl)-3H-4-oxo-thieno(3,2-d)pyrimidine monohydrochloride (8)

A mixture of compound (7) (5.8 g, 10.24 mmol) in 50 ml of a 6% solution of hydrogen chloride in methanol was stirred at 20°C for 20 minutes and 120 ml of diethyl ether was then added to gradually form a white precipitate. After one hour, the crystalline C-nucleoside monohydrochloride (8) was filtered and washed with ether to give 2.56 g (88%) of the desired product, mp 211-214°C.

Anal. Calcd: C: 41.19, H: 4.08, N: 8.73, S: 9.99
Found: C: 41.59, H: 4.10, N: 8.65, S: 9.85
Structure was confirmed by NMR spectroscopy

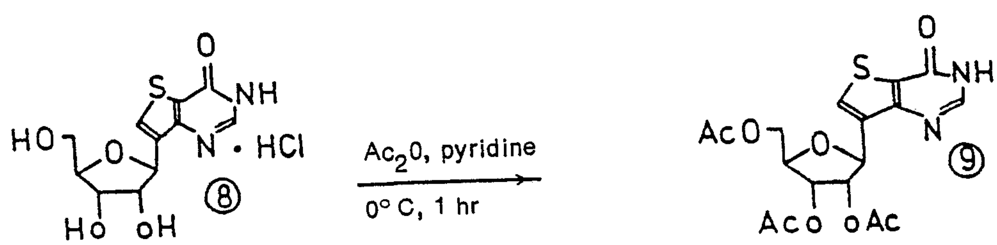


7-(2′,3′,5′-tri-0-acetyl-β-D-ribofuranosyl)-3H-4-oxo-thieno(3,2-d)pyrimidine (9)

To a solution of compound (8) (1.5 g, 5.28 mmol) in anhydrous pyridine (5 ml) was added acetic anhydride (5 ml) at 0°C. After one hour the mixture was partitioned between chloroform (100 ml) and water (100 ml). The organic layer was washed with water (100 ml), dried over anhydrous $Na_2SO_4$ and evaporated to dryness *in vacuo.* The residue containing (9) was purified by chromatography on a silica gel column with chloroform-methanol (40:1) to give 1.61 g (75%) of the triacetate (9) as a colorless syrup. The structure was confirmed by elemental analysis and NMR spectroscopy.

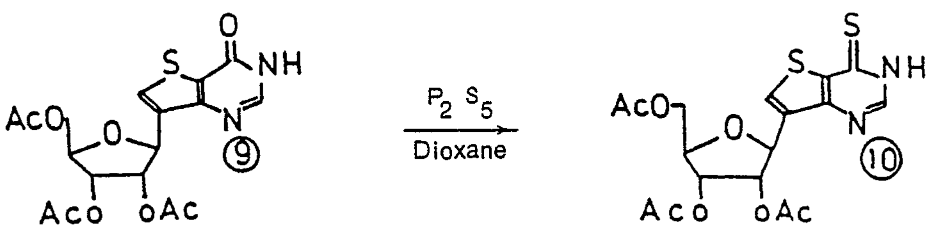


Thieno [3,2–*d*] pyrimidine
analog of Thioniosine

7-(2′,3′,5′-Tri-0-acetyl-β-D-ribofuranosyl)-3H-4-thiono-thieno(3,2-d)pyrimidine (10)

To a solution of (9) (3 g, 7.31 mmol) in 40 ml of dry dioxane heated to reflux was added 3 g of phosphorous pentasulfide in portions (200 mg each) over a period of 1.5 hours. Heating was continued until thin layer chromatography (chloroform/methanol: 10/1) indicated that the reaction was completed. The solvent was removed *in vacuo* and the residue containing (10) was purified by chromatography on a column of silica gel (chloroform/methanol: 20/1) to give the thieno-pyrimidine thione (10) in pure form (2.62 g, 84%) as a foam.

The structure was confirmed by NMR spectroscopy.

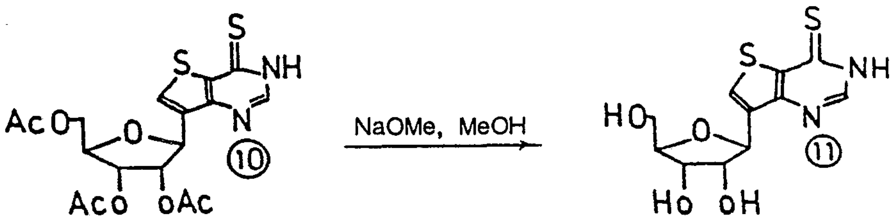


7-(β-D-ribofuranosyl)-3H-4-thiono-thieno(3,2-d)pyrimidine ⑪

A solution of triacetate ⑩ (2.02 g, 4.74 mmol) in 40 ml of 0.1 N sodium methoxide in methanol was stirred at 20°C for 1 hour. The final solution was neutralized with IRC—50 (H+) ion exchange resin, filtered, and the filtrate evaporated to dryness *in vacuo.* The residue was dissolved in water and the aqueous solution washed with chloroform. The aqueous layer was lyophilized to give 1.36 g (95%) of pure 7-(β-D-ribofuranosyl)-3H-4-thiono-thieno(3,2-d)pyrimidine ⑪ as a powder.

The structure was confirmed by NMR spectroscopy

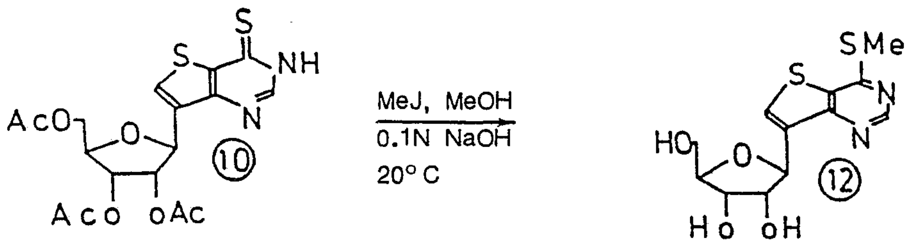


Thieno 3,2–*d* pyrimidine analog of Methylthio purine riboside

7-(β-D-Ribofuranosyl)-4-methylthio-thieno(3,2-d)pyrimidine ⑫

To a solution of triacetate ⑩ (350 mg, 0.82 mmol) in a mixture of 3 ml of methanol and 3.3 ml of methyl iodide was added 10 ml of 0.1 N aqueous sodium hydroxide and the reaction mixture was stirred at room temperature for one hour. During this period, the product desired ⑫ precipitated. This was collected by filtration, washed with methanol then chloroform to afford 255 mg (98%) of 7-(β-D-ribofuranosyl)-4-methylthio-thieno(3,2-d)pyrimidine ⑫ as a crystalline (white needles) material. One recrystallization from boiling methanol afforded the analytical sample.

mp 226-228°C,

| | | | | |
|---|---|---|---|---|
| Anal Calcd: | C: 45.84, | H: 4.48, | N: 8.90, | S:20.39 |
| Found: | C: 45.89, | H: 4.51, | N: 8.88, | S: 20.25 |

The structure was confirmed by NMR spectroscopy.

TABLE 1

In vitro activity ($ID_{50}$'s in µg/ml) of C-nucleosides in mouse and human leukemic cell lines

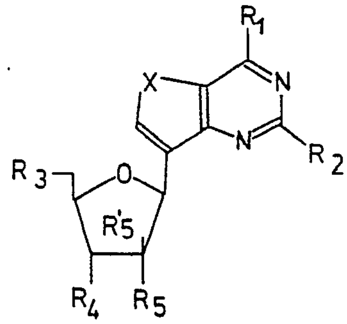

| | | P—815 | L—1210 | RAJI | ALL—CCRF—CEM | HL—60 |
|---|---|---|---|---|---|---|
| X=NH<br>$R_2$=H<br>$R_3$=$R_4$=<br>$R_5$=OH,<br>$R_5'$=H | $R_1$=OH, $R_2$=H | >10(0%) | >100 | >100 | >100 | >100 |
| | $R_1$=SH, $R_2$=H | 5;6 | — | — | — | — |
| | $R_1$=SMe, $R_2$=H | 0.3 | — | — | — | — |
| | $R_1$=$NH_2$, $R_2$=H | 0.001 | 0.0008 | 0.002 | 0.0008 | 0.0003 |
| X=S<br>$R_2$=H<br>$R_3$=$R_4$=<br>$R_5$=OH,<br>$R_5'$=H | $R_1$=OH, $R_2$=H | 2.5 | 3.3 | 4.2 | 0.9 | 0.4 |
| | $R_1$=SH, $R_2$=H | 0.5 | 0.6 | 0.9 | 1.6 | 0.4 |
| | $R_1$=SMe, $R_2$=H | 0.03 | 0.07 | 0.03 | 0.006 | 0.008 |
| | $R_1$=$NH_2$, $R_2$=H | 0.0003 | 0.0006 | 0.002 | 0.0005 | 0.0005 |

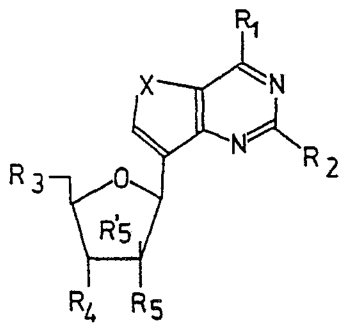

TABLE 2

In vivo activity of C-nucleosides in mice

| C-nucleoside | Line | Schedule (MG/KG) @ Dose | % ILS |
|---|---|---|---|
| X=NH | L—1210/O | QD × 5, $D_1$ @ 0.5 | 9.1 |
| $R_1$=$NH_2$, $R_2$=H | L—1210/O | Q4D × 3, $D_1$ @ 0.25 | 17.0 |
| $R_3$=$R_4$=$R_5$=OH<br>$R_5'$=H | L—1210/MP | Q4D × 3, $D_1$ @ 0.4 | 71.8 |
| | P—815/ARA C | Q4D × 3, $D_1$ @ 0.7 | 30.8 |
| | P—815/ARA C | Q4D × 3, $D_1$ @ 0.4 | 19.8 |
| X=S<br>$R_1$=$SCH_3$, $R_2$=H | P—815/O | QD × 2/Q2D × 3 @ 60 | 71.4 |
| $R_3$=$R_4$=$R_5$=OH<br>$R_5'$=H | P—815/O | Q4D × 4, $D_1$ @ 40 | 56.3 |

**Claims**

1. Beta-glycosyl C-nucleoside compound of the formula

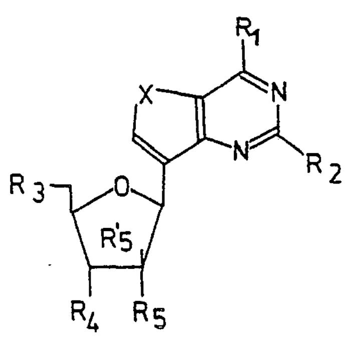

wherein

X is S or O

$R_1$ is 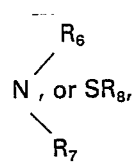, or $SR_8$, or $OR_8$

wherein $R_6$, $R_7$ and $R_8$ are independently selected from H or alkyl of 1 to 6 carbon atoms; or

$R_2$ is H,
$R_3$ is OH,
$R_4$ is OH,
$R_5$ is OH or H,
$R'_5$ is H.

2. The compound of claim 1 designated 7-(β-*D*-Ribofuranosyl)-4-amino-thieno(3,2-*d*)pyrimidine.

3. The compound of claim 1 designated 7-(2'Deoxy-β-*D*-ribofuranosyl)-4-amino-thieno(3,2-*d*)pyrimidine.

4. The compound of claim 1 designated 7-(β-*D*-Ribofuranosyl)-4-amino-furo(3,2-*d*)pyrimidine.

5. The compound of claim 1 designated 7-(2'Deoxy-β-*D*-ribofuranosyl)-4-amino-furo(3,2-*d*)pyrimidine.

6. In a method for preparing the beta-glycosyl C-nucleoside compound of one of the proceeding claims, the steps of

(a) providing a blocked sugar β-ribofuranosyl C-glycoside substituted by β-dimethylaminoacrylonitrile;

(b) hydrolyzing the dimethylamino group to a hydroxyl group under conditions which do not effect the blocking groups;

(c) forming a five membered heterocyclic ring either by reaction of said hydroxyl group with N-alkyl- or aminoacetonitrile followed by a ring closure, or by mesylation of said hydroxyl group substituting the mesyl group for an oxygen or sulfur containing group suitable to effect ring closure;

(d) separating alpha and beta isomers;

(e) forming a pyrimidine ring fused with said five-membered ring, from the beta isomer; and

(f) unblocking the sugar.

**Patentansprüche**

1. Beta-Glykosyl C-Nukleosid-Verbindung mit der Formel

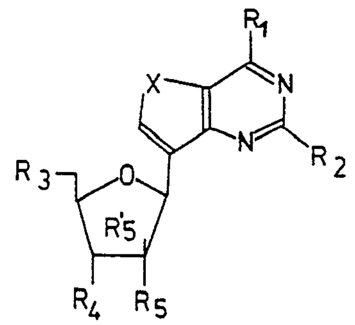

wobei

X S oder O ist

$R_6$
/
$R_1$ N, oder $SR_8$, oder $OR_8$ ist,
\
$R_7$

wobei $R_6$, $R_7$ und $R_8$ unabhängig ausgewählt ist aus H oder einem Alkyl mit 1 bis 6 Kohlenstoffatomen; oder wobei

$R_2$ H ist,
$R_3$ OH ist,
$R_4$ OH ist,
$R_5$ OH or H ist,
$R'_5$ H ist.

2. Substanz nach Anspruch 1, nämlich 7-(β-*D*-Ribofuranosyl)-4-amino-thieno(3,2-*d*)pyrimidin.

3. Substanz nach Anspruch 1, nämlich 7-(2'Desoxy-β-*D*-ribofuranosyl)-4-amino-thieno(3,2-*d*)pyrimidin.

4. Substanz nach Anspruch 1, nämlich 7-(β-*D*-Ribofuranosyl)-4-amino-furo(3,2-*d*)pyrimidin.

5. Substanz nach Anspruch 1, nämlich 7-(2'Desoxy-β-*D*-ribofuranosyl)-4-amino-furo(3,2-*d*)pyrimidin.

6. In einem Verfahren zur Herstellung der β-Glykosyl C-Nucleosid-Substanz nach einem der vorherigen Ansprüche, mit den Schritten

(a) Liefern eines mit Schutzgruppen versehenen ("blocked") Zukker-β-Ribofuranosyl-C-Glykosides, das durch β-Dimethylaminoacrylonitril substituiert ist;

(b) Hydrolisieren der Dimethylamino-Gruppe zu einer Hydroxyl-Gruppe unter Bedingungen, die die mit Schutzgruppen versehenen Gruppen nicht angreifen;

(c) Bilden eines fünfgliedrigen heterocyclischen Ringes entweder durch die Reaktion der Hydroxyl-Gruppe mit N-Alkyl- oder Aminoacetonitril gefolgt durch einen Ringschluß, oder durch Mesylation der

besagten Hydroxyl-Gruppe, indem die Mesyl-Gruppe an die Stelle von einer Sauerstoff oder Schwefel enthaltenden Gruppe tritt, die geeignet ist, einen Ringschluß zu bewirken.

(d) Auftrennen der Alpha- und Beta-Isomere.

(e) Bilden eines Pyrimidinringes, der mit dem fünfgliedrigen Ring verschmolzen ist, aus dem Beta-Isomer und

(f) Entfernen der Schutzgruppen ("unblocking") vom Zucker.

**Revendications**

1. Composé de type béta-glycosyl C-nucléoside, de formule

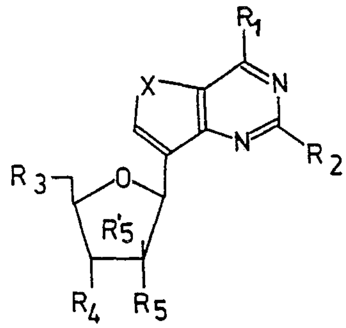

dans laquelle

X est S ou O

$R_6$
/
$R_1$ est N, ou $SR_8$, ou $OR_8$
\
$R_7$

où $R_6$, $R_7$, $R_8$ sont choisis indépendamment parmi H ou un alkyle de 1 à 6 atomes de carbone; ou

$R_2$ est H,
$R_3$ est OH,
$R_4$ est OH,
$R_5$ est OH ou H,
$R'_5$ est H.

2. Composé suivant la revendication 1, qui est la 7-(β-*D*-ribofuranosyl)-4-amino-thiéno(3,2-*d*)pyrimidine.

3. Composé suivant la revendication 1, qui est la 7-(2'déoxy-β-*D*-ribofuranosyl)-4-amino-thiéno(3,2-*d*)pyrimidine.

4. Composé suivant la revendication 1, qui est la 7-(β-*D*-ribofuranosyl)-4-amino-furo(3,2-*d*)pyrimidine.

5. Composé suivant la revendication 1, qui est la 7-(2'déoxy-β-*D*-ribofuranosyl)-4-amino-furo(3,2-*d*)pyrimidine.

6. Dans un procédé pour la préparation du composé béta-glycosyl C-nucléoside suivant l'une des revendications précédentes, les opération de

(a) prépartion d'un sucre bloqué de type β-ribofuranosyl C-glycoside substitué par β-diméthylamino-acrylonitrile;

(b) hydrolyse du groupe diméthylamino en un groupe hydroxyle dans des conditions qui n'affectent pas les groupes de blocage;

(c) formation d'un anneau hétérocyclique à cinq éléments, par réaction dudit groupe hydroxyle avec un groupe N-alkyl- ou aminoacétonitrile suivie par une fermeture du cycle, ou bien par mésylation dudit groupe hydroxyle substituant le groupe mésyle à la place d'un groupe contenant de l'oxygène ou du soufre, de manière à effecteur la fermeture du cycle;

(d) séparation des isomères alpha et béta;

(e) formation d'un anneau pyrimidine fusionné avec ledit anneau à cinq éléments à partir de l'isomère béta; et

(f) élimination du blocage du sucre.